# EUROPEAN PATENT SPECIFICATION

(11) **EP 1 487 369 B1**
(45) Date of publication and mention of the grant of the patent: **03.05.2017**
(21) Application number: 03716593.3
(22) Date of filing: 13.03.2003
(51) Int. Cl.: A61B 5/154, A61B 5/155, A61B 5/15, A61M 1/02

(54) **BIOLOGICAL FLUID SAMPLING APPARATUS**
GERÄT ZUR ENTNAHME VON BIOLOGISCHEN FLÜSSIGKEITEN
APPAREIL DE PRELEVEMENT DE FLUIDE BIOLOGIQUE

(30) Priority: 14.03.2002 US 364314 P; 24.10.2002 US 279251; 15.11.2002 US 295151
(43) Date of publication of application: 22.12.2004
(73) Proprietor: Fenwal, Inc., Lake Zurich, IL 60047 (US)
(72) Inventor: MATHIAS, Jean-Marie, B-1428 Lillois (BE); RONDEAU, Georges, B- Braffe (BE); PETERSON, Grant, A., Chicago, IL 60645 (US); BOMBINO, Armando, Crystal Lake, IL 60014 (US); JOIE, Michel, B-4030 Erange (BE)
(74) Representative: Geary, Stephen
(86) International application number: PCT/US2003/007945
(87) International publication number: WO 2003/078964

(56) References cited:
- WO-A2-01/58507
- DE-U1- 20 108 230
- US-A- 3 395 696
- US-A- 3 687 296
- US-A- 4 655 764
- US-A- 4 932 418
- US-A- 4 943 283
- US-A- 5 084 034
- US-A- 5 259 843
- US-A- 5 372 143
- US-A- 5 496 301
- US-A- 5 620 008
- US-A- 5 620 008
- US-B1- 6 520 948
- US-B2- 6 387 086

## Description

The present invention relates to sampling apparatus suitable for collecting or taking samples of biological fluid, such as blood or blood components, from fluid circuit assemblies employed in the collection, separation, storage or processing of biological fluids.

### BACKGROUND OF THE INVENTION

Disposable fluid circuit assemblies are typically used for collecting, storing, separating and/or other processing of biological fluids, such as blood from a donor, patient or other source. They are commonly preassembled, presterilized and fully disposable for safety and convenience. These assemblies may include plastic tubing, containers, valves, flow control modules and the like for controlling fluid flow through the assembly. When employed on the collection or processing of blood and blood components, these assemblies typically include a venipuncture needle for insertion into the arm of a donor or patient. The needle is usually attached to one end of a flexible plastic tube which provides a flow path for the blood to the rest of the fluid circuit assembly. The other end of the plastic tube may be attached, either directly or indirectly, to one or more plastic bags or containers for collecting the withdrawn blood or a component of blood, such as concentrated red cells or platelets, or plasma. Such fluid circuit assemblies may be employed in manual blood collection procedures, where whole blood is collected from a donor for later off-site processing, or in automated procedures, where the fluid circuit is mounted on a reusable device, such as a centrifuge or other separator, that automatically controls flow of blood or components through the assembly. When used for blood collection, these fluid circuit assemblies are commonly called blood sets or apheresis sets.

The fluid circuit assembly may also include a sampling sub-unit to provide for collection of a sample of blood or blood component, which can be used for testing. It is known to use pierceable junctions in the fluid circuit to allow the user to extract a sample at the desired location. This has associated with it, however, a risk of accidental needle puncture as well as undesirable pooling of the fluid at the location of the sample port.

It also is well known to collect samples of biological fluid, such as blood or blood components, in vials or tubes that are employed in combination with a tube holder or receiver that communicates directly with a patient or donor or communicates with a sample container that is part of a larger fluid processing circuit assembly. The receiver typically has an internal needle that punctures a resilient septum on the end of the sample tube when the tube is inserted into the receiver. A vacuum within the sample tube draws blood into the sample tube. One of the most common sample tubes is the Vacutainer™ brand sample tube sold by Becton-Dickinson of Franklin Lake, New Jersey.

As shown in U.S. Patent No. 5,496,301, it is also known to use a sample bag or pouch that is connected by a length of tubing to a sample tube holder for cooperating with vacuum sample tube, such as the Vacutainer™ sample collection tube. The holder includes a cylindrical shield and an internal needle within an elastomeric sheath for cooperation with the Vacutainer™ tube.

The arrangement illustrated in the above patent, however, is not as conductive to ease of manufacture or economy of packaging as may be desired. Also, in addition to other apparent shortcomings, it requires manual manipulation, such as inversion and the like to retrieve a sample and may not allow for easy and rapid withdrawal of the entire sample contained in the sample bag.

Document US4932418 discloses the preamble of claim 1.

### SUMMARY OF THE INVENTION

The present invention, is embodied, in one aspect, in a tube holder or receiver assembly for cooperating with a sample collection tube, such as a Vacutainer™ vacuum sample collection tube. In accordance with this aspect, the receiver or tube assembly includes a piercing member or needle assembly including one end for piercing the end of a sample tube and another end, which may be a non-piercing end or also may have a piercing member, and a generally cylindrical housing with a distal end engageable with the needle assembly and a proximal end that is adapted to receive a sample collection tube. To protect the interior of the housing and help avoid accidental contact with the piercing member, the assembly includes a cover movable between a closed position covering the proximal end and an open position opening the proximal end for receipt of a sample collection tube. The cover is preferably hinged to the cylindrical housing and the hinge is disposed to move the cover to either an open or closed position preferentially to an intermediate position.

The sample tube receiver may be used by itself or in combination with other apparatus, such as a sample container or pouch, an intravenous needle assembly or a fluid circuit assembly for manual or automated processing of biological fluid such as blood or blood components.

In still a further aspect of the present invention, a sampling device receiver is attached to tubing leading to a container, which may be a sample receiving container or a larger container, such as a blood bag or plasma collection bottle. In this embodiment, the sample device may be directly attached to the tubing or may be directly attached to a connection fitting, such as a V-site or Y-site, that is associated with the tubing.

### BRIEF DESCRIPTION OF DRAWINGS

Figure 1 is a plan view of a biological fluid circuit assembly in the form of disposable blood collection or processing set including a sampling apparatus employed in the present invention.
Figure 2 is a top view of the receiver or holder assembly of Figure 1 with the cover in an open position.
Figure 2A is a top view of a sample tube receiver or holder with a modified cover design.
Figure 3 is a side view of the receiver assembly of Figure 2 with the cover in an open position.
Figure 3A is an enlarged side view of the hinge arrangement of the receiver in Figures 2 and 2A.
Figure 4 is a perspective view of the receiver assembly of Figure 2 with the cover in the open position.
Figure 5 is a partial side view of the receiver assembly with a portion of the outer sidewall broken away to show the holder assembly interior in cross-section.
Figure 5A is a side cross-sectional view of a sample tube receiver in the present invention, without the needle assembly.
Figure 5B is a side cross-sectional view of the sample tube receiver of Figure 5A with a needle assembly, partially removed, in place.
Figure 6 is a plan view of a manual fluid circuit assembly embodying sampling apparatus of the present invention.
Figure 7 is a plan view of sampling apparatus of the present invention shown in Figure 6, including a container and a sample tube receiver.
Figure 8 is a plan view of an alternative embodiment of sampling apparatus of the present invention.
Figure 9 is a plan view of a further alternative sampling apparatus of the present invention.
Figure 10 is a plan view of a still further alternative sampling apparatus of the present invention.
Figure 11 is a vertical cross-section view of sampling apparatus taken along line 11-11 of Figure 7 and illustrating the sampling apparatus within an outer package.
Figure 11A is a plan view of the sampling apparatus of 7, showing the inlet tubing coiled around the container and receiver to provide a convenient and low profile packaging arrangement.
Figure 12 is a plan view of the sampling apparatus of Figure 7 with the receiver cover in the open position to receive a sample tube.
Figure 13 is a plan view of showing insertion of a sample tube, such as a Vacutainer™ vacuum sample tube, into the receiver of Figure 12.
Figure 14 is a perspective view of a fluid circuit assembly mounted on a reusable device for automated blood processing, and embodying the present invention.
Figures 15-16 are cross-sectional and top views, respectively, of the piercing end of a prior art needle.
Figures 17-19 are side, top and bottom views, respectively, of the piercing end of another prior art needle.
Figure 20 is a side view of a piercing needle particularly useful in the sampling apparatus of the present invention.
Figure 21 is a top view of the needle of Figure 20.
Figure 22 is a bottom view of the needle of Figure 20.
Figure 23 is perspective view of the needle of Figure 20.
Figure 24 is a perspective view of another needle that may have application in the sampling apparatus of the present invention.
Figure 25 illustrates the surface of a pierceable septum after repeated piercing by a prior art needle.
Figure 26 illustrates the surface of a pierceable septum after repeated piercing by the needle of Figures 20-23.
Figure 27 is a plan view of a sampling apparatus attached directly to a Y-site located in a fluid collection line attached to a plasma pooling or collection bottle.

### DETAILED DESCRIPTION OF THE DRAWINGS

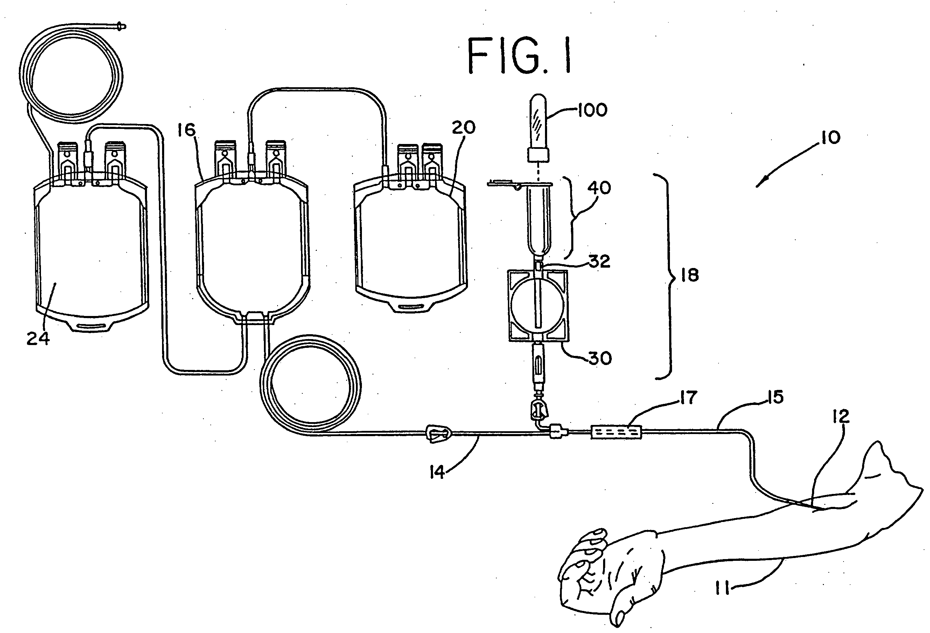

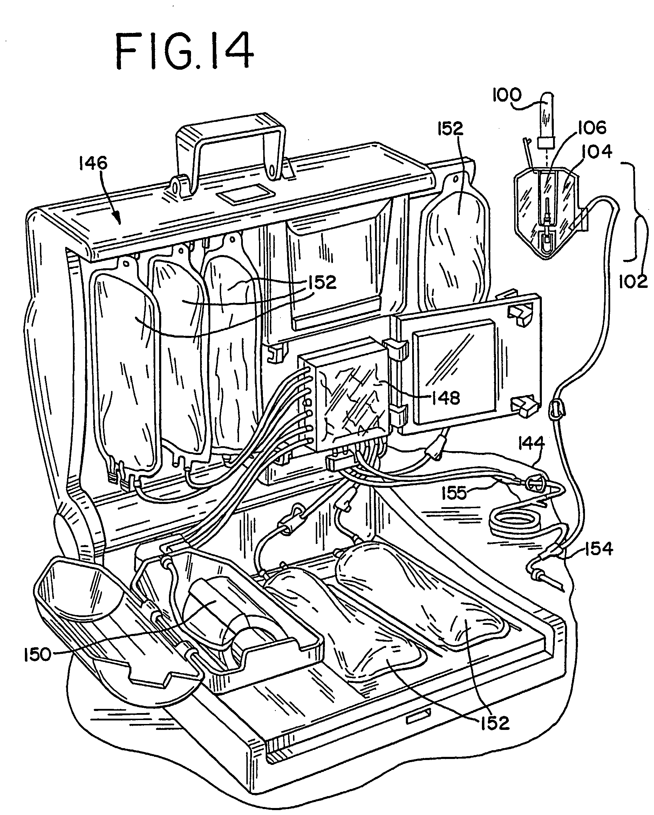

Turning first to Figures 1-5, there is shown, among other things, a holder or receiver (holder and receiver are used interchangeably herein) assembly 40 employed in the present invention. The holder assembly 40 may be part of a pre-sterilized fluid circuit assembly such as a blood collector and processing set 10 for the manual collection of blood from a donor 11, shown in Figure 1. Alternatively, holder assembly 40 may be part of an apheresis processing set for the automated collection of blood and blood components as illustrated in Figure 14.

It will also be appreciated that the holder assembly of the present invention may be provided as a "stand-alone" device (i.e., not used with a processing set of the type shown or described above) for "direct" withdrawal of blood from a donor or patient. An example of what is meant by a "stand-alone" device is described in U.S. Patent No. 5,372,143. Another embodiment of a "stand-alone" receiver of the present invention includes a "double-needled" holder assembly where one of the needles is directly inserted into the vein of a donor or patient. For purposes of the following discussion, however, the holder or receiver assembly shall be described in conjunction with a sample collection container, which may be part of a larger fluid circuit assembly, such as the disposable blood collection and processing set of Figure 1.

As shown in Figure 1, the illustrated disposable blood collection/processing set 10 may include a needle such as venipuncture needle 12, and plastic tubings 14 and 15 extending from needle 12 to a collection container such as a flexible plastic container 16. A needle protector 17 may also be provided for retraction and storage of needle 12 after use.

The blood processing set 10 may include a single blood collection container 16 or, more preferably, as shown in Figure 1, may include additional containers such as 20 and 24 for separation, storage or other processing of the collected blood or blood components. In accordance with one particular, non-limiting embodiment, the disposable processing set 10 may include a sampling sub-unit 18, including sampling container or pouch 30.

In a preferred embodiment, sampling sub-unit 18 includes a receiver or holder assembly 40 of the present invention. Receiver assembly 40 may be pre-attached to blood sampling tube 32 of sampling container or pouch 30, thereby establishing flow communication between the holder assembly and the pouch interior. Details of the blood collection and blood sampling procedures using the above-described sets are described in U.S. Applications Serial Nos. 09/364,628 and 09/492,060, incorporated by reference.

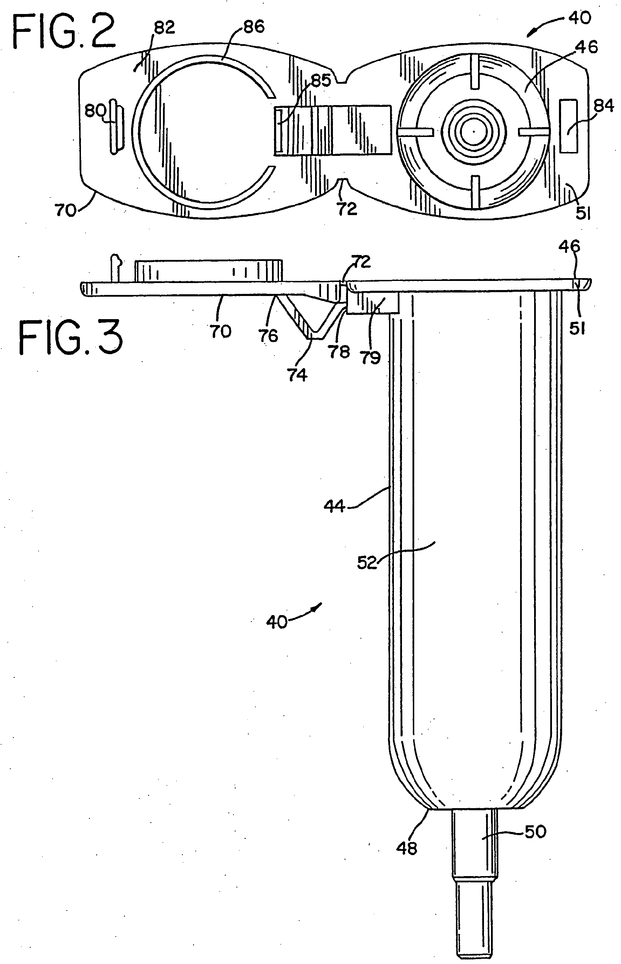

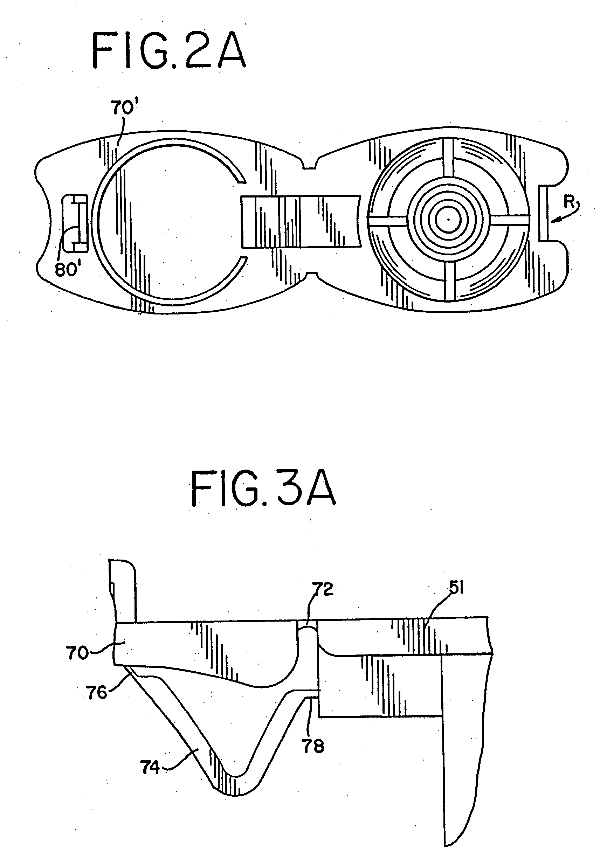

In one embodiment (shown in Figure 3), receiver assembly 40 includes an elongated hollow cylindrical housing 44, although other shapes or geometries may also be employed. Housing 44 includes a proximal end 46 and distal end 48. Housing 44 is open (and/or openable) at its proximal end 46 and is adapted for receiving therethrough a blood collection tube 100, such as a Vacutainer™ vacuum collection container.

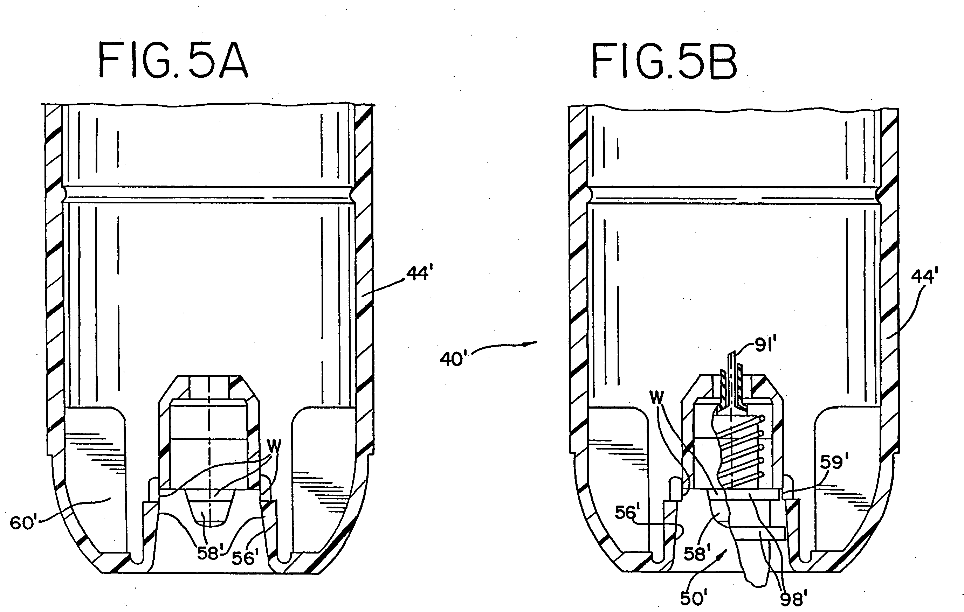

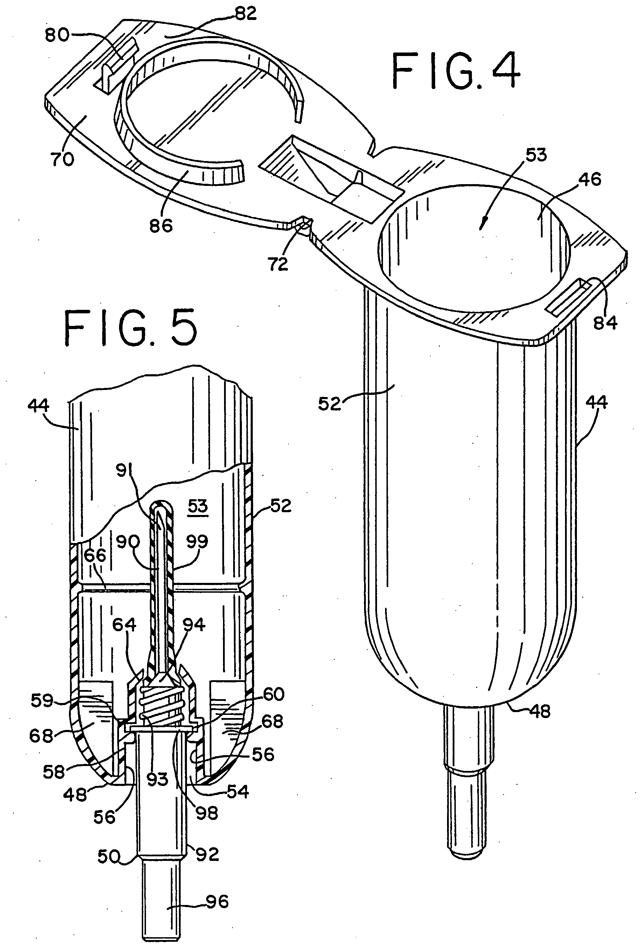

In a preferred embodiment (described in greater detail below), housing 44 of holder assembly 40 includes a cover 70 for closing the open proximal end 46 of the housing 44. The distal end 48 of the housing 44 is generally closed except for external access through a needle subassembly 50 (described in greater detail below with reference to Figure 5) secured to the housing 44 at the distal end 48.

Housing 44 may be made of any suitable, plastic material that can be injection molded and sterilized by known forms of sterilization, such as autoclaving (steam sterilization) or radiation. A preferred, autoclavable plastic material is polypropylene. Where holder assembly 40 is sterilized by electron beam or gamma radiation, suitable materials may include polystyrene. Of course, still other materials known to those of skill in the art may also be used.

As shown in Figure 3, in one embodiment, cylindrical housing 44 may optionally include flange 51 at or near the open proximal end 44. Sidewall 52 of housing 44 extends from flange 51 to distal end 48. At the distal end 48, as seen in Figure 5, side wall 52 is interrupted by axial through bore 54, centered within the distal end, and adapted to receive needle subassembly 50.

As best seen in Figure 5, axial bore 54 is defined by an interior wall 56. The housing 44 and needle subassembly 50 preferably have interlocking surfaces for fixedly holding the needle assembly in the through bore. The interlocking surfaces may be of a variety of different arrangements. As seen in Figure 5, wall 56 may include an interior annular shoulder or ledge 59 and a radially inwardly extending rib 58 located proximally from the distal end 48. The inwardly extending ledges or ribs 58 and 59 are spaced apart from each other to provide an annular slot 60 in the interior wall 56.

As further shown in Figure 5, the diameter of distal axial bore 54 is smaller in the area proximal to second inwardly extending ledge 59, than in the area distal to the first inwardly extending ledge 58. In the illustrated embodiment, the diameter of axial bore 54 is further narrowed by inwardly radially inclined wall portion 64.

As shown in Figure 5, housing 44 may also include an inner, radially inward collar or ring 66. Collar 66 may be provided as a ledge or projection extending from the interior surface of sidewall 52 into the housing interior 53. Collar 66 provides a means for frictionally retaining the needle protector 17 (with a used venipuncture needle retracted therein), which may be inserted into housing 44 after blood collection procedure is completed, as generally described in U.S. Application Serial No. 09/442,210. Housing 44 may also include a stop surface for engaging the end of a blood sampling tube 100. In the illustrated embodiment stop surfaces are provided by the proximal ends of ribs 68 in the distal end of the housing interior 53.

Housing 44 of needle holder assembly 40 preferably includes a cover, such as cap 70 for opening and closing proximal end 46 of the cylindrical housing. Cap 70 may be separately provided or, more preferably, may be attached to housing 44, as shown in the figures. In one embodiment (see Figures 3 and 4), cap may be a "flip cap" attached to the flange 51 by hinge 72. Hinge 72 may be formed by reducing (during the injection molding process) the amount of plastic material and, thus, the thickness in the section between cap 70 and flange 51, allowing for easy bending along the hinge.

As shown in Figures 3 and 3A, cap 70 may also be provided with spring type or plastic "living hinge" closure member 74. One end of closure member 74 is attached to the cap 70 along thin web 76 while the other end 78 of closure member 74 is attached to base 79 of flange 51 along thin web 78. Closure member 74 biases the cap to either an open position or a closed position preferentially to an intermediate position, so as to allow for "one handed" and, in fact, "one-fingered" opening and closing of cap 70. With a single flick of the thumb or finger, the technician can open or close the cap as necessary. Closure member 74 causes cap 70 to either snap open or snap closed, and tends to move the cap away from an intermediate position to the open or closed position preferentially to an intermediate position. In other embodiments, cap 70 may be provided as a tethered cap (i.e., tethered to housing 44), a cap that slides over the open proximal end, a cap that rotates between an open and closed position, or other arrangement. A peel off seal or cover, sealed adhesively or otherwise bonded to the open end of housing 44 could also be used to provide a sterile barrier over the open end of housing 44 until a sample is required.

As further shown in Figures 2 and 3, cap 70 may include a latch 80 on the inner surface 82 of cap 70. When cap 70 is in the closed position, latch 80 is received within slot 84 of flange 50. Cap 70 may also include centering ring 86 for centering cap 70 over open proximal end 46 and, thereby ensuring proper closure. Optional gap 85 between spring 74 and cap 70 allows for venting of housing interior 53 during, for example, steam sterilization.

Turning now to the needle subassembly 50, it will be appreciated that needle subassembly may be integrally joined to housing 44 as a one-piece arrangement or attached by adhesive or melt bonding or, more preferably, the needle subassembly may be separately formed and adapted for interference fit to housing 44 by interlocking surfaces. Needle subassembly 50 may include a proximal piercing end 90 and distal non-piercing end 92. Although referred to as a needle assembly, it is absolutely necessary that the member for a needle be used to pierce the septum of the sample tube. A blunt cannula or the like could also be used with those sample tubes that would accommodate such. Also, where holder assembly is a "stand-alone" type assembly intended for direct use with a donor or patient, needle assembly 40 may include a distally-pointed needle, for example, a double-ended needle where both the proximal and distal ends include piercing ends. Optionally, the proximal and/or distal ends of the piercing member could have the well-known blunt cannula configuration to provide greater safety against accidental needle sticks, as described above.

With reference to the embodiment shown in Figure 5, first proximal piercing end 90 includes piercing member 91 attached to hub 94. Piercing member 91 may be a hollow needle or cannula made of stainless steel or other rigid metal or plastic. The opposite facing distal non-piercing end 92 preferably includes a luer 96 with an internal fluid path but a blunt cannula or needle could also be used if desired. The internal fluid path of the distal end is in fluid communication with the hollow interior of the proximal needle (piercing member) 91 via a through bore in the needle hub.

As an alternative to snap attachment, the portion 93 of the body of needle subassembly between luer 96 and hub 94 may, optionally, be threaded to allow attachment to housings having a threaded axial bore. However, in a preferred embodiment, needle subassembly 50 is not screwed into housing 44, but is instead press-fit into housing 44. In one press-fit arrangement, needle subassembly 50 may include an outwardly extending radial ring 98 for press fit engagement with housing 44. Specifically, as needle subassembly 50 is advanced into axial bore 54, ring 98 is captured within slot 60. Further movement in the proximal or distal directions is prevented by inwardly extending rib or ledge 58 and 59, thus providing a secure attachment of needle subassembly 50 to housing 44. These structures could be reversed, with the needle hub having a pair of spaced ribs defining a slot therebetween and the housing having an annular rib for snap fit into the slot. Other structures, such as detents, latches and the like, could also be used for snap fit assembly. A secure fit is partially desired so as to avoid blood leakage.

Needle 91 is preferably enclosed within a flexible, resilient protective sheath such as a rubber (latex) sleeve, or more preferably a polyisoprene or other non-latex sleeve 99. Sleeve 99 is located over needle 91 and hub 94. Hub 94 may include an outwardly extending ring (not shown), to provide a tight fit between hub 94 and the distal end of the sleeve, and thereby hold sleeve 99 in place. Other techniques may also be used for attaching the sleeve to the needle hub 94 and/or the cylindrical housing 44 -- such as adhesive bonding, friction fit, clamping and the like. In another embodiment, sleeve 99 is loosely placed over needle 91 and hub 94. A loose fitting or vented sleeve may be preferred (as compared to a sleeve that is stretched over the hub) in that it is presently believed to be less susceptible to oxidation during sterilization by electron beam or gamma sterilization. In the illustrated embodiment sleeve 99 is held in place by radially extending wall 64.

When a vial is inserted into housing 44, the end or septum of the vial forces the needle through the proximal end of the sheath 99 and into the vial. As the vial continues to be inserted, the sheath is forced distally to a collapsed configuration. When the vial is withdrawn, the resilient sheath 99 preferably resumes its position over the needle although such may not be required in sampling apparatus intended for one-time use only.

With a receiver assembly of the type described above, the technician can easily open and close the open proximal end of the holder assembly, as necessary. For example, once a sample has been collected (in the collection tube), the technician can, with a simple flick of his finger against the cap 70, close the housing 44. With another flick of the thumb or finger, the technician can open the housing to allow for insertion of the next tube. Thus, it will be appreciated that the easy manipulation of cap 70 provided by the present invention allows for a rapid, fluid and substantially uninterrupted sampling motion, while protecting the technician from accidental contact with the needle between sample draws, and from contact with blood residing in the holder interior. The holder assembly can later be reopened and utilized as a secure receptacle for a used venipuncture needle/needle protector, as described above. Fast and uninterrupted withdrawal is important because blood collected near the point of withdrawal from the donor or patient may not contain anticoagulant and it is important to be able to collect the sample before coagulation begins.

Figures 2A and 5A show alternative and preferred configurations of the cover or cap 70', needle subassembly 50' and housing 44'. As shown in Figure 2A, the upper flange 51' of the housing 44' has a recessed edge area R. The cover 70' is sized to extend over and beyond the recessed edge area when in the closed position, with the latch 80' engaged to the flange at the recessed edge. The edge of the cover overlying the recess R is slightly concave. This provides a visible indicator to the user that the user's thumb or finger can be placed in this location to raise the cover.

Figures 5A and 5B show an alternative and preferred housing and needle assembly arrangement. The housing 44' is comparable to the housing 44 of Figure 5 except that instead of a continuous internal rib 58, there are 4 spaced-apart rib arc segments 58', each of which extends a short distance around the inside of interior 56'. A small, generally rectangular opening or window W (formed by the molding apparatus) extends through interior wall immediately above each rib segment 58'. The windows allow the molding apparatus to better form the rib segments without deformation of the material when the mold opens.

The needle subassembly 50' in this embodiment is similar to that described earlier except that it has two radial flanges or rings 98'. Referring to Figure 5B, upper ring 98', as seen in Figure 5B, fits in snap engagement between the rib segments 58' and internal shoulder or ledge 59'. To prevent the escape of any blood that may leak from the inside of housing 44' through the windows W, the lower ring 98' is engaged in a fluid-tight interference fit against the surface of the interior wall 56'.

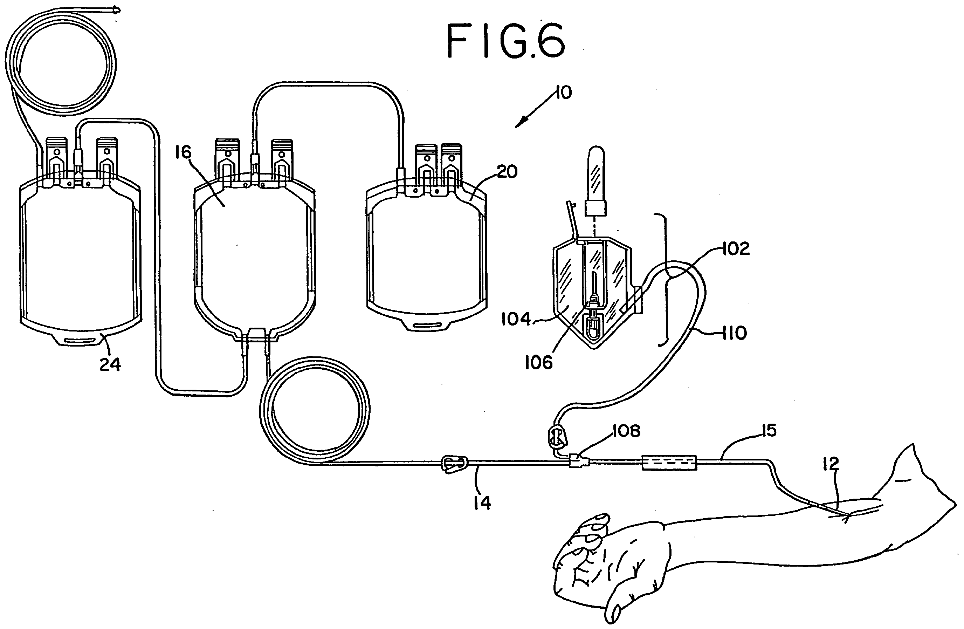

An alternative arrangement employing the present invention is shown in Figure 6, which depicts a fluid circuit assembly in form of a blood processing set, like that shown in Figure 1, with the exception of the sampling sub-unit. As shown in Figure 6, fluid circuit assembly is a blood processing set principally intended for manual collection and processing of whole blood from a donor or other source. The blood processing set 10 shown there includes a needle 12, tubing 14 and 15 for conveying whole blood to a blood receiving container 16 and additional containers 20 and 24 connected, via tubing, to collection container 16, for receiving blood components after a separation process has been carried out. To provide for sampling of the whole blood received from the donor or other source, a sampling sub-unit 102 is provided as an integral part of the fluid circuit assembly. The sampling sub-unit 102 includes a sample-receiving container 104 and a sample tube receiver or holder 106. The container receives whole blood through tubing 110 which is attached to the blood inlet line 14 at junction 108.

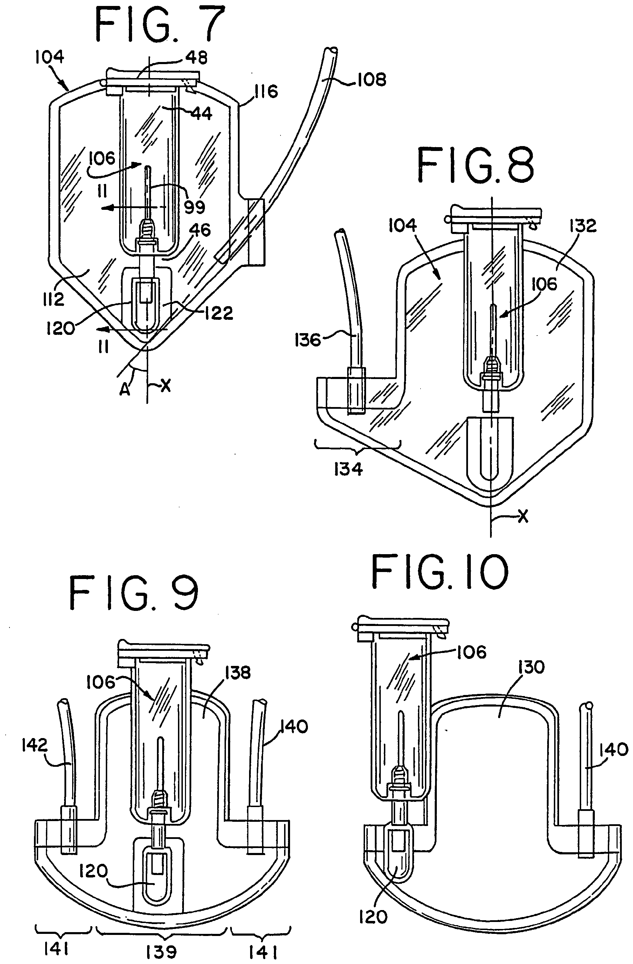

The sampling sub-unit and alternative embodiments thereof are shown in Figures 7-11. Turning to Figure 7, the sampling apparatus of the present invention, as illustrated in this embodiment, has advantages with respect to both handling and packaging. As seen in Figures 7-11, the sample-receiving container is preferably, but not exclusively, a flexible container in the form of a pouch formed by peripherally sealing together two facing flexible plastic sheets 112 and 114. The sheets, which may be of polyvinylchloride or other suitable material, either with or without plasticizer, that can withstand gamma or E-beam irradiation sterilization or autoclave sterilization, are peripherally sealed together along a peripheral edge 116 to define a generally closed internal chamber for receiving blood or other biological fluid through tubing 108. The container 104 may be of any suitable shape, but in the preferred embodiment, the container walls are shaped to direct the incoming blood (or other fluid if used in a non-blood collection fluid circuit) to a selected location in the interior chamber. In the embodiment shown in Figure 7, the container is adapted for holding in a generally vertical position, as shown in Figure 7, and fluid is directed into the lowermost region of the container by the inclined peripheral edges in the lower portion of the container. For purposes of reference and description in discussing its vertical position, the container is shown as having an imaginary vertical axis X.

To assist and accommodate the vertical disposition of the container 104, inlet tubing 108, as illustrated in Figure 7, extends through the peripheral edge 116 in a direction which is at an acute angle A with respect to the imaginary vertical axis of the container.

To remove samples from the container, the sampling sub-unit 102 includes the sample tube holder or receiver 106 of the present invention, which is carried by a wall of the container and preferably directly attached to a wall of the container. The sample tube receiver or holder 106 is essentially identical to that previously described in Figures 2-5, and includes a generally cylindrical housing 44 with proximal and distal ends 46 and 48, and having a piercing member such as a needle 99 mounted therewithin for cooperation with a sample collection container or vial 100.

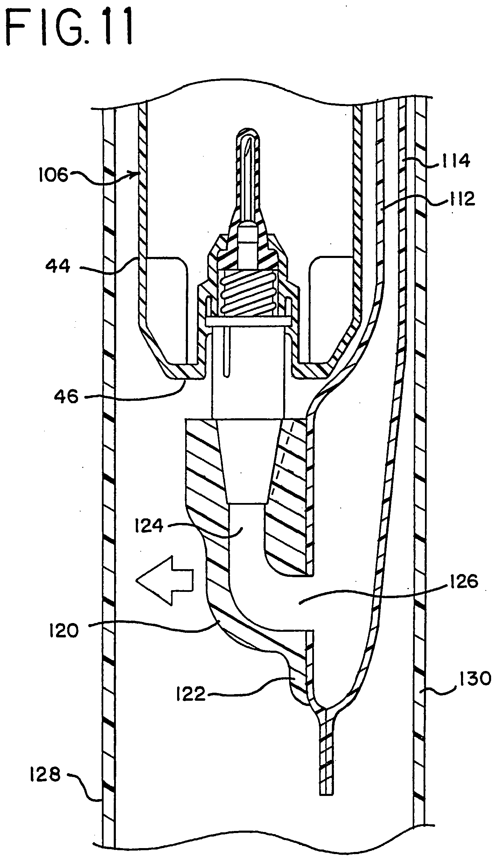

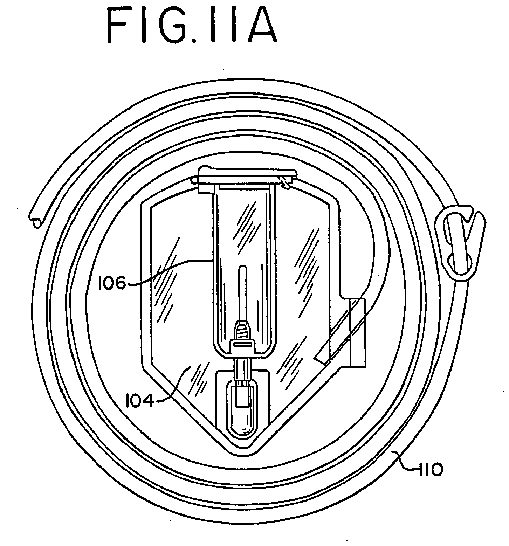

In the embodiment shown in Figure 7, the distal end member 46 of the receiver assembly is attached, such as by ultrasonic welding, bonding or the like to a mounting member 120 that has a peripheral flange 122 for attachment to the flexible plastic sheet of the container. As best seen in Figure 11, the mounting member 120 includes an interior passageway 124 that allows the sample tube receiver to communicate directly with a sample exit opening 126 located in the wall of the container. The exit opening 126 from the container is preferably located in the area where fluid collects and where is fluid is directed to collect by the walls of the container. In the embodiment shown in Figures 7 and 11, this is lowermost region of the container when the container is in the vertical disposition. To direct the blood to the lowermost region, the lower peripheral edges of the container, as seen in Figure 7, are inclined to channel or funnel the fluid into the lowermost region. This arrangement better assures that substantially the entire sample collected within the container may be withdrawn if desired and has other advantages as well.

The sampling apparatus shown in Figure 7 has the additional benefit of a compact, lay-flat configuration for packaging. As can be seen in at least Figures 7, 8 and 9, the sample tube holder or receiver 106 does not extend substantially beyond the peripheral edge of the container to which it is mounted, and substantially overlies the container. In other words, the receiver 106 is entirely or substantially within the "footprint" of the container. This aspect of the combined container and receiver cooperates with the angular direction of the tubing 108 to provide a convenient lay-flat configuration of the container and sample tube receiver, with the inlet tubing 108 coiled around it. For example, as seen in Figure 11, the sample tube receiver and container provide a low profile packaging arrangement between upper and lower walls 128 and 130 of an outer package, and the coiled tubing fits easily with the low profile package, as illustrated in Figure 11A.

Additional embodiments with differing configurations of the sample receiving container are shown in Figures 8-10. As shown in Figure 8, the sample receiving container 132 is shaped similarly to that of Figure 7, with the exception that the container includes a laterally extending region 134 at which the inlet tube 136 is attached in a generally vertical direction. Thus, the interior chamber of the container also includes, in part, a laterally extending region, into which the inlet tube 136 communicates. The sample tube receiver or holder 106 employed in Figure 8 is essentially identical to that shown in Figure 7, and is attached, by a mounting member 120, to a wall of the container so that it communicates with the interior chamber in the lowermost region of the container where the blood is directed.

Another embodiment of the sampling apparatus is shown in Figure 9. The sample container 138 of Figure 9 is likewise made by peripherally sealing together a pair of flexible plastic sheets. In this embodiment as in the other embodiments discussed, the container could have one or more rigid walls, and be made of injection molded plastic, if it were so desired.

The container 138 in Figure 9 has a generally large central region 139 and a pair of opposed lateral regions 141 into which fluid flow tubing 140 and 142 communicate. In this embodiment, for example, tubing 140 could connect the container directly to the blood flow inlet line, and tubing 142 could be used for withdrawing blood from the container so that the sampling apparatus is not at the end of a fluid communication line, but is in-line in a fluid flow passageway that leads to another part of the fluid circuit.

Also intended for holding in a vertical disposition, the container 138 in Figure 9 has a generally arcuate or upwardly concave lower peripheral edge, so that blood is directed into the lowermost region. The container wall includes an exit opening in the lowermost region to permit blood flow from the container to a sample tube located in the receiver 106. The receiver is mounted, via mounting member 120, in the same manner as described above with respect to Figures 7 and 11.

Figure 10 shows another sampling apparatus embodying certain aspects of the present invention, which is identical to Figure 9, except that the sample tube receiver is mounted in one of the laterally offset extending regions of the container, and the container does not have an outlet tube.

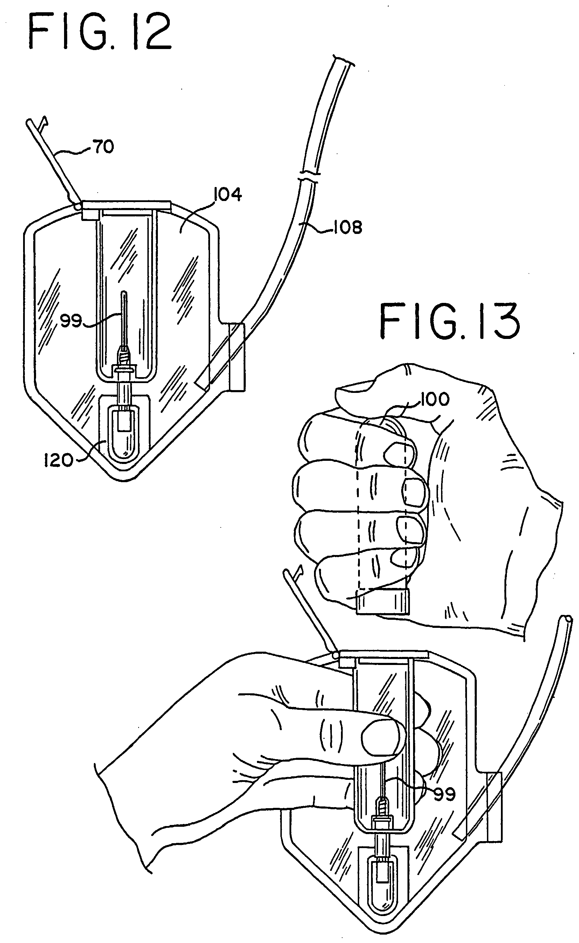

Figures 12-13 show how the apparatus of the present invention may be employed in taking a sample from a fluid circuit assembly. Having collected a fluid sample through tubing 108 into the container 104, the container and receiver are held in a generally vertical position, with the proximal end of the receiver facing upwardly, and the cover or cap of the container is opened. As explained earlier, the living hinge arrangement moves the cap to an open position with a simple flick of the thumb to raise the cap. The blood collection vial or tube 100 is then inserted downwardly into the receiver, as shown in Figure 13. The blood collection tube includes a septum of latex or other suitable material at the bottom of the tube that is pierced by the needle 99 located within the cylindrical housing of the receiver. The interior of the blood collection tube typically is a vacuum, which tends to draw contents from the sample outlet in the lowermost region of the container upwardly through the mounting member and needle and into the collection tube. This procedure, which requires minimal manipulation of the container and blood sample and employs a vertical insertion of the sample tube, has the further advantage of reducing hemolysis of the blood or blood component. Repeated samples can be taken simultaneously or periodically as usage requires. When the sample has been taken, the cap on the tube receiver is closed to protect the interior needle from accidental engagement by the user.

As described earlier, in addition to possible stand-alone applications, the present invention may be used in both manual and automated fluid circuit assemblies. Figures 1 and 6 illustrate typical manual fluid circuit assemblies for blood and blood component collection. Figure 14 illustrates a fluid circuit assembly specifically intended for automated collection and processing and employing the sample apparatus of the present invention. Except for the sample apparatus of the present invention, the fluid circuit assembly and automated collection device shown in Figure 14 are as described in detail in U.S. Patent No. 6,325,775. The fluid circuit, generally at 144, shown in Figure 14 is intended for use with a portable, suitcase-size processing device 146. Without repeating all of the disclosure set forth in the above-identified patent, the disposable fluid circuit assembly shown in Figure 14 includes a fluid circuit control module 148 which is adapted for mounting onto the device 146, and which has associated controllers for controlling the direction and flow through the fluid circuit. The fluid circuit assembly may also include a separation device, generally at 150, through which anticoagulated whole blood flows for separation into one or more blood components, such as red cells, platelets or plasma. The fluid circuit assembly may also include miscellaneous containers 152 for receiving blood or blood components or for containing anticoagulant, saline, or other liquids required during the blood processing.

In accordance with the present invention, blood sampling apparatus or sub-unit 102 is attached to the fluid circuit assembly at a Y-site or V-site junction 154 in the line leading from the donor access needle, preferably before anticoagulant is added to the whole blood at junction 155, although it may be attached at any other location in the fluid circuit where there is a need or desire to sample the fluid at that location. In all other respects, the sampling apparatus 102 is identical to that described earlier in connection with Figures 7 and 11-13.

Turning now to a description of the needle 99 employed in the sample receiver, it should first be noted that the needle may be used to repeatedly puncture the rubber or latex septum of a sample tube, and it is desirable that the needle not unduly damage the septum or generate particulate matter.

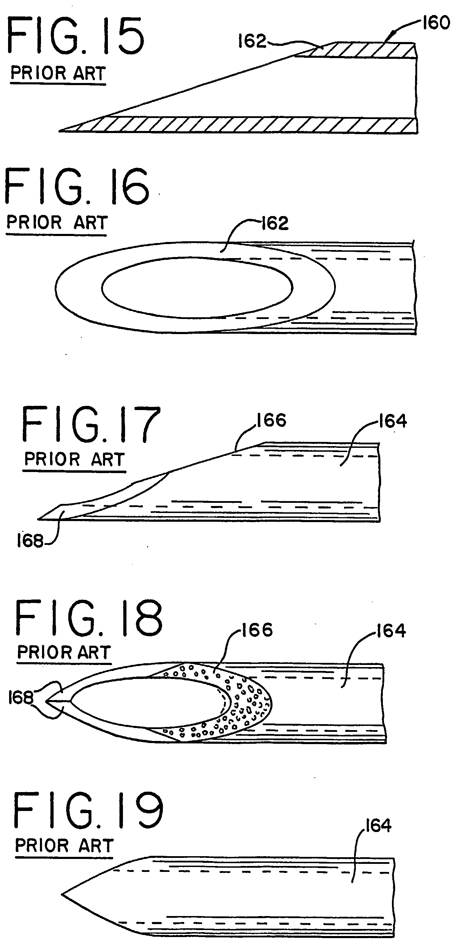

Figures 15-19 show prior needle tip designs that have been employed in various medical applications. Figures 15-16 show the sharpened end of a stainless steel needle 160, employing a straight bevel facet to form the needle tip. Figure 15 is a cross-sectional view of such a needle, and shows a straight or plain beveled surface 162. Figure 16 shows the same needle and the elliptical facet surface 162 from a top view.

Figures 17-19 illustrate another prior needle 164. The sharpened tip of needle 164 has a flat or straight primary bevel grind surface 166 in a proximal region of the needle tip, and outwardly angled (or downwardly diverging) secondary bevel facets 168 leading from the flat bevel to the distal most end of the needle tip. The primary bevel is subjected to microsandblasting, a technique known in the field, which erodes the edges of the primary bevel to make the heel of the needle opening more rounded.

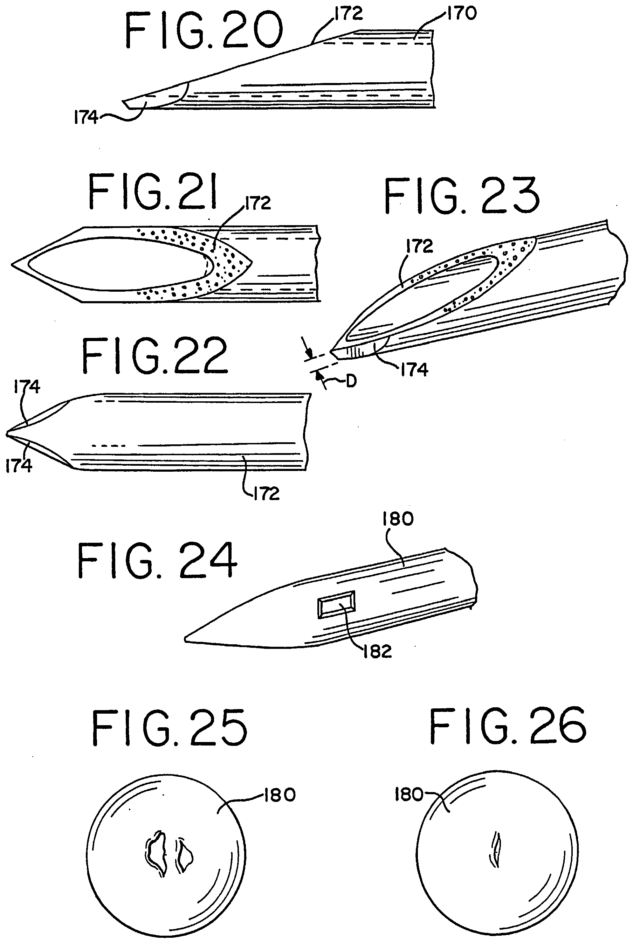

Figures 20-23 show the tip configuration of a needle 170 as preferably used in the fluid sampling apparatus of the present invention. The needle tip there includes a generally flat primary needle grind to generate facet 172 in a proximal region of the needle tip. A pair of inwardly angled (or downwardly converging) secondary side bevel grind surfaces or facets 174 are next formed sequentially at the distal most end of the needle tip. These secondary facets may be formed by rotating the needle shaft in one direction for a selected angle greater than 90° and less than 180°, grinding one secondary facet and then rotating the needle back to the start position and then in the other direction at the same angular displacement, where the other secondary facet is ground. The two secondary facets preferably extend not more than about 30% of the length of the primary bevel. The intersection of the internal surface of the hollow needle with the proximal portion of the primary bevel (the heel) can be a very sharp edge or blade that is responsible for coring of the septum or other material pierced by the needle. To reduce the potential for coring, the proximal portion of the primary bevel, at least in the area of the heel, is preferably microsandblasted to smooth the sharp edges and reduce the potential for coring. The side bevel facets 174 converge at the tip to form the distal most tip 176 of the needle at an interior location spaced a distance D from the side surface of the needle shaft.

Tests of the needle 170 show substantially improved results relative to septum destruction or particle generation. Figure 26 shows a typical collection tube septum 180 repeatedly punctured or pierced six times with the needle 170. The puncture area is visible, but limited and confined to generally one location. Figure 25 shows such a septum repeatedly pierced six times with the prior art needle of Figures 15-16. This needle tends to enter the septum at different locations with each puncture, and the tearing and destruction of the septum is more severe and is not localized.

Figure 24 shows an alternative needle 180 which may also provide for improved septum piercing in which the needle tip is generally plain and closed, except for a lateral rectangular aperture 182 formed in the side of the needle wall for fluid communication after the septum has been pierced by the closed point of the needle shaft.

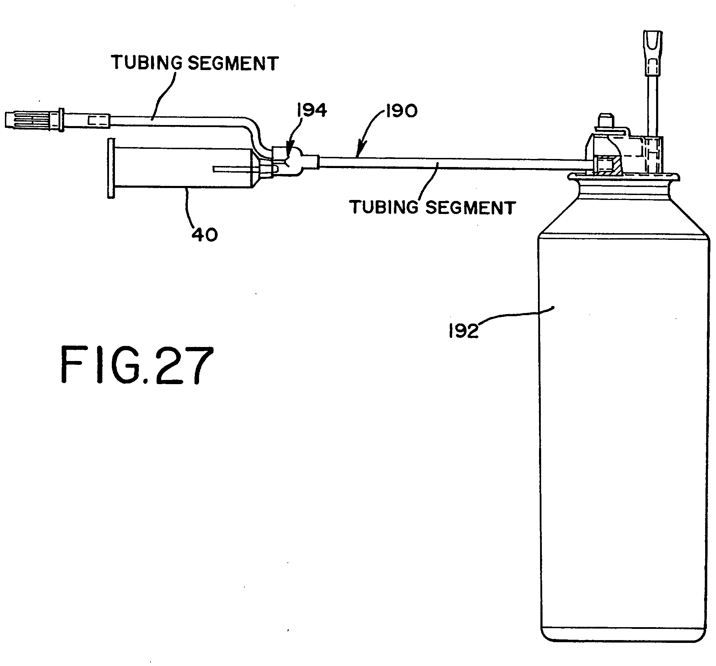

Figure 27 shows a sample tube holder or receiver assembly 40 as part of a fluid flow system that may be used with a blood processing device or system, such as that known as the Baxter Autopheresis-C® separator from Baxter Healthcare Corporation of Deerfield, Illinois. Such a separator and the associated disposable tubing system is shown in more detail in U.S. Patents Nos. 5,194,145 and 5,135,667. The present invention is, of course, not limited to use with Baxter systems, but may also be used in connection with separation apparatus available from others, such as Haemonetics Corporation of Raintree, Massachusetts, or Gambro, Inc. of Lakewood, Colorado.

The Autopheresis-C® separator is often used for collecting blood plasma from donors. The plasma is typically collected in a rigid or semi-rigid plastic container or bottle that is attached to the disposable tubing set used on the Autopheresis-C® device.

The apparatus shown in Figure 27 is particularly suited for such an application. In that apparatus, the receiver assembly 40 is attached to tubing 190 that extends between the separator tubing set and a plasma collection bottle or container 192. The tubing 190 is attached, typically aseptically, to the fluid flow system of the separator. After the bottle 192 is filled with plasma, the tubing may be sealed and severed.

In accordance with the present invention, the receiver assembly 40 as previously described is attached substantially directly to the tubing 190 to allow samples to be taken of fluid, such as plasma, flowing through the tubing. Preferably, the receiver assembly 40 is attached directly to a connector 194 located in the fluid flow line. The connector 194 is typically of one-piece molded plastic construction, employing any suitable material, such as polyvinylchloride. It may be of any appropriate shape such as Y-shape, V-shape, T-shape or other shape which allows fluid to be withdrawn from the tubing.

As shown in Figure 27, the connector is a typical Y-site or Y-connector, as often found on disposable medical fluid tubing sets. One leg of the Y forms a port that is attached to a segment of the tubing 190 extending to the collection bottle 192 and another leg of the Y forms a port that is attached to another segment of the tubing 190. For use in this arrangement, the receiver assembly 40 preferably terminates in a projection, such as a standard hollow luer type male fitting, suitable for insertion into a third leg or port of the Y-connector 194. The receiver is preferably permanently attached to the connector by solvent or ultrasonic bonding although other means for attachment, both permanent and temporary may be suitable.

Assembled in the above manner, a convenient and ergonomically efficient fluid system is provide for attachment to an apheresis system as described above, with reduced risk of contamination. In the past, blood or plasma collection centers have had to maintain a variety of products or parts and have been required to devote the time to assemble the various parts for every procedure. With the invention as shown in Figure 25, a complete pre-assembled sterile set may be provided for ready attachment to the desired apheresis system without the distraction of or the potential for error associated with collecting and assembling the various parts and pieces. Further, as shown in the attached drawing, there is reduced likelihood of cross-contamination, which may occur if the same sampling apparatus is used to take samples from different apheresis procedures. With the present invention, in a preferred version, the sample tube holder or receiver 40 is permanently attached to the tubing associated with a particular collection set or procedure.

Although the present invention has been described in terms of the illustrated embodiments, the intended scope of the invention is as set forth in the appended claims and the illustrated embodiments in this description are intended as an illustration and not intended as a limitation to the subject matter set forth in the claims.

## Claims

1. A blood collection tube holder assembly comprising:
a needle assembly comprising a proximal end and a distal end, the proximal end terminating in a piercing member (91) and the distal end terminating in a non-piercing blunt cannula (96), the needle assembly further comprising a hub (94) located between said proximal and distal ends, said hub including a radially extending wall (64); wherein said piercing member (91) is enclosed within a flexible sleeve(99) said sleeve having a distal end attached to said radially extending wall (64) of said hub to hold said sleeve in place;
a generally cylindrical housing (44) including a distal end and a proximal end and a sidewall extending between the proximal and distal ends, the proximal end adapted to receive a blood collection tube and the distal end comprising a through-bore (54) defined by an interior wall engageable with the needle assembly;
wherein the housing includes an interlocking surface and the needle assembly includes an interlocking surface for fixedly holding the needle assembly in the housing through-bore by interlocking engagement of the interlocking surfaces,
wherein the interlocking surface of the housing comprises an inwardly extending annular ledge (59) and an inwardly extending annular rib (58) axially spaced from said ledge to define an annular slot (60) therebetween and the interlocking surface of the needle assembly comprises an outwardly extending radial ring (98) adapted for snap-fit engagement in slot (60) to secure said needle assembly to said housing (44).

2. The holder assembly according to claim 1, wherein the needle assembly comprises two outwardly extending radial rings, being an upper radial ring and a lower radial ring.

3. The holder assembly according to claim 2, wherein the upper radial ring fits in snap engagement in the slot (60) between the inwardly extending annular ledge (59') and an inwardly extending annular rib (58').

4. The holder assembly according to claim 2 or claim 3, wherein the lower radial ring (98') is engaged in an interference fit against the surface of the interior wall (56').

5. The holder assembly according to claim 1, further comprising a cover (70) movably associated with the proximal end of the cylindrical housing and movable between a closed position covering the proximal end and an open position opening the proximal end.

6. The holder assembly of claim 5 wherein said cover (70) is attached to said housing (44') by a hinge.

7. The holder assembly according to claim 6 further comprising a closure member that has a first end attached to the cover and a second end attached to the housing, the closure member being disposed to hold the cover in either the open or closed position and to move the cover preferentially from an intermediate position to the open or closed position.

## Patentansprüche

1. Halterbaugruppe für Blutentnahmeröhren, umfassend:
eine Nadelbaugruppe, welche ein proximales Ende und ein distales Ende umfasst, wobei das proximale Ende in einem stechenden Element (91) endet und das distale Ende in einer nicht stechenden stumpfen Kanüle (96) endet, wobei die Nadelbaugruppe ferner eine Nabe (94) umfasst, welche zwischen dem proximalen und dem distalen Ende angeordnet ist, wobei die Nabe eine radial verlaufende Wand (64) enthält; wobei das stechende Element (91) in einer flexiblen Hülle (99) eingeschlossen ist, wobei die Hülle ein distales Ende hat, welches an der radial verlaufenden Wand (64) der Nabe angebracht ist, um die Hülle an Ort und Stelle zu halten;
ein im Wesentlichen zylindrisches Gehäuse (44), welches ein distales Ende und ein proximales Ende und eine Seitenwand enthält, welche zwischen dem proximalen und dem distalen Ende verläuft, wobei das proximale Ende dafür ausgebildet ist, eine Blutentnahmeröhre aufzunehmen und das distale Ende eine Durchgangsbohrung (54) umfasst, welche durch eine innere Wand begrenzt ist, die mit der Nadelbaugruppe in Eingriff bringbar ist;
wobei das Gehäuse eine Verriegelungsfläche enthält und die Nadelbaugruppe eine Verriegelungsfläche enthält, um die Nadelbaugruppe durch verriegelndes Eingreifen der Verriegelungsflächen fest in der Durchgangsbohrung des Gehäuses zu halten,
wobei die Verriegelungsfläche des Gehäuses einen einwärts verlaufenden ringförmigen Ansatz (59) und eine einwärts verlaufende ringförmige Rippe (58) enthält, welche von dem Ansatz axial beabstandet ist, um einen ringförmigen Schlitz (60) dazwischen zu bilden, und die Verriegelungsfläche der Nadelbaugruppe einen auswärts verlaufenden radialen Ring (98) umfasst, welcher für ein einrastendes Eingreifen in dem Schlitz (60) ausgebildet ist, um die Nadelbaugruppe an dem Gehäuse (44) zu sichern.

2. Halterbaugruppe nach Anspruch 1, wobei die Nadelbaugruppe zwei auswärts verlaufende radiale Ringe enthält, welche ein oberer radialer Ring und ein unterer radialer Ring sind.

3. Halterbaugruppe nach Anspruch 2, wobei der obere radiale Ring in einrastendem Eingriff in dem Schlitz (60) zwischen dem einwärts verlaufenden ringförmigen Ansatz (59') und einer einwärts verlaufenden ringförmigen Rippe (58') steht.

4. Halterbaugruppe nach Anspruch 2 oder Anspruch 3, wobei der untere radiale Ring (98') in Festsitz gegen die Fläche der inneren Wand (56') in Eingriff steht.

5. Halterbaugruppe nach Anspruch 1, ferner umfassend eine Abdeckung (70), welche bewegbar mit dem proximalen Ende des zylindrischen Gehäuses verbunden ist und zwischen einer geschlossenen Stellung, welche das proximale Ende abdeckt und einer offenen Stellung, welche das proximale Ende öffnet, bewegbar ist.

6. Halterbaugruppe nach Anspruch 5, wobei die Abdeckung (70) durch ein Scharnier an dem Gehäuse (44') angebracht ist.

7. Halterbaugruppe nach Anspruch 6, ferner umfassend ein Schließelement, welcher ein an der Abdeckung angebrachtes erstes Ende und ein an dem Gehäuse angebrachtes zweites Ende hat, wobei das Schließelement so angeordnet ist, dass es die Abdeckung entweder in der offenen oder in der geschlossenen Stellung hält und die Abdeckung vorzugsweise von einer mittleren Stellung in die offene oder in die geschlossene Stellung bewegt.

## Revendications

1. Ensemble de support de tube de collecte de sang comprenant :
un ensemble d'aiguille présentant une extrémité proximale et une extrémité distale, l'extrémité proximale se terminant par un élément de perforation (91) et l'extrémité distale se terminant par une canule émoussée non perforante (96),
l'ensemble d'aiguille comprenant en outre un manchon (94) situé entre lesdites extrémités proximale et distale, ledit manchon comportant une paroi s'étendant radialement (64) ; dans lequel ledit élément de perforation (91) est contenu dans un fourreau flexible (99),
ledit fourreau présentant une extrémité distale fixée sur ladite paroi s'étendant radialement (64) dudit manchon afin de maintenir ledit fourreau en place ;
un boîtier sensiblement cylindrique (44) comportant une extrémité distale et une extrémité proximale et une paroi latérale s'étendant entre les extrémités proximale et distale, l'extrémité proximale étant adaptée de manière à recevoir un tube de collecte de sang et l'extrémité distale comprenant un orifice traversant (54) défini par une paroi intérieure pouvant être couplée à l'ensemble d'aiguille ;
dans lequel le boîtier comporte une surface de verrouillage et l'ensemble d'aiguille comporte une surface de verrouillage destinée à maintenir fermement l'ensemble d'aiguille dans l'orifice traversant de boîtier par couplage de verrouillage des surfaces de verrouillage,
dans lequel la surface de verrouillage du boîtier comprend un rebord annulaire s'étendant vers l'intérieur (59) et une nervure annulaire s'étendant vers l'intérieur (58) espacée axialement par rapport audit rebord afin de définir une fente annulaire (60) entre les deux, et la surface de verrouillage de l'ensemble d'aiguille comprend une bague radiale s'étendant vers l'extérieur (98) adaptée de manière à assurer un couplage par encliquetage dans la fente (60) afin de fixer l'ensemble d'aiguille sur ledit boîtier (44).

2. Ensemble de support selon la revendication 1, dans lequel l'ensemble d'aiguille comprend deux bagues radiales s'étendant vers l'extérieur, constituées par une bague radiale supérieure et une bague radiale inférieure.

3. Ensemble de support selon la revendication 2, dans lequel la bague radiale supérieure est couplée par encliquetage dans la fente (60) entre le rebord annulaire s'étendant vers l'intérieur (59') et une nervure annulaire s'étendant vers l'intérieur (58').

4. Ensemble de support selon la revendication 2 ou 3, dans lequel la bague radiale inférieure (98') est couplée par assemblage forcé contre la surface de la paroi intérieure (56').

5. Ensemble de support selon la revendication 1, comprenant en outre un couvercle (70) associé de manière à pouvoir se déplacer avec l'extrémité proximale du boîtier cylindrique et pouvant être déplacé entre une position fermée recouvrant l'extrémité proximale et une position ouverte libérant l'extrémité proximale.

6. Ensemble de support selon la revendication 5 dans lequel ledit couvercle (70) est fixé sur ledit boîtier (44') par une charnière.

7. Ensemble de support selon la revendication 6 comprenant en outre un élément de fermeture qui comporte une première extrémité fixée sur le couvercle et une seconde extrémité fixée sur le boîtier, l'élément de fermeture étant disposé de manière à maintenir le couvercle dans l'une ou l'autre des positions ouverte ou fermée et à déplacer le couvercle préférentiellement à partir d'une position intermédiaire vers la position ouverte ou fermée.
